# EUROPEAN PATENT SPECIFICATION

(11) **EP 4 547 144 B1**
(45) Date of publication and mention of the grant of the patent: **05.08.2026**
(21) Application number: 23755574.3
(22) Date of filing: 26.07.2023
(51) Int. Cl.: A61B 50/30, A61B 50/00

(54) **MEDICAL DEVICE PACKAGING WITH MICROORGANISM RESISTANT CHANNELS**
VERPACKUNG FÜR MEDIZINISCHE VORRICHTUNG MIT MIKROORGANISMENRESISTENTEN KANÄLEN
EMBALLAGE DE DISPOSITIF MÉDICAL AVEC CANAUX RÉSISTANTS AUX MICRO-ORGANISMES

(30) Priority: 02.08.2022 US 202263370150 P; 08.05.2023 US 202363500764 P
(43) Date of publication of application: 07.05.2025
(73) Proprietor: Edwards Lifesciences Corporation, Irvine, CA 92614 (US)
(72) Inventor: UPADHYAYA, Sameer Dinmanishanker, Irvine, California 92614 (US); ROTH, Arti Prasad, Irvine, California 92614 (US); RAJPARA, Vipul P., Irvine, California 92614 (US)
(74) Representative: Venner, Julia Ann
(86) International application number: PCT/US2023/071019
(87) International publication number: WO 2024/030798

(56) References cited:
- WO-A2-2023/167982
- US-A- 3 754 700
- US-A- 4 057 144
- US-A- 5 922 162

## Description

### RELATED APPLICATION(S)

This application claims priority to U.S. Provisional Patent Application No. 63/370,150, filed on August 2, 2022, and entitled MEDICAL DEVICE PACKAGING WITH MICROORGANISM RESISTANT CHANNELS, and priority to U.S. Provisional Patent Application No. 63/500,764, filed on May 8, 2023, and entitled MEDICAL DEVICE PACKAGING WITH MICROORGANISM RESISTANT CHANNELS.

### BACKGROUND

### Field

The present disclosure generally relates to the field of medical device packaging.

### Description of Related Art

Medical devices are often packaged in containers to protect the medical devices and/or maintain a sterilized state of the medical devices during manufacturing/distribution/point of use or at other times. For example, a medical device can be packaged and sterilized into a container to prevent the medical device from being contaminated by microorganisms (e.g., bacteria, virus, pathogens etc.) and/or from being damaged while being moved from one location to another.

US 3,754,700 describes surgical pouches having a back wall of sheet polyethylene and a front wall of sheet polyethylene with a header piece of sterilizable, surgical kraft paper or other gas permeable, sheet material having bacteria holdout properties, wherein the instrument contained within the pouch may be sterilized after the pouch is sealed.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention.

Described herein are devices, methods, and/or systems for packaging medical devices in a manner that facilitates gaseous or other forms of sterilization of the medical devices within the packaging and prohibits/impedes microbial ingress/penetration.

For purposes of summarizing the disclosure, certain aspects, advantages, and/or features are described. It is to be understood that not necessarily all such advantages may be achieved in accordance with any particular example. Thus, the disclosed examples can be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes. In addition, various features of different disclosed examples can be combined to form additional examples, which are part of this disclosure. Throughout the drawings, reference numbers may be reused to indicate correspondence between reference elements.
Figure 1 illustrates packaging that includes a gas-permeable seal(s) to facilitate gas sterilization or another gaseous process to treat a medical device disposed therein in accordance with one or more examples.
Figure 2 illustrates details of the gas-permeable seal(s) of Figure 1 that can be implemented in accordance with one or more examples.
Figure 3 illustrates configurations/implementations of the gas-permeable seal of Figure 1 with multiple paths/channels in accordance with one or more examples.
Figure 4 illustrates configurations/implementations of the gas-permeable seal of Figure 1 with a single path/channel through the gas-permeable seal in accordance with one or more examples.
Figure 5 illustrates a configuration/implementation of the gas-permeable seal of Figure 1 with an at least partially parallel path through the seal with respect to a top edge of the packaging in accordance with one or more examples.
Figure 6 illustrates a configuration/implementation of the gas-permeable seal of Figure 1 that includes openings positioned on different sides/edges of the packaging in accordance with one or more examples.
Figures 7A and 7B illustrate packaging that includes pores/holes configured in an offset manner on multiple packaging members/membranes to facilitate a channel that allows for gas and prohibits entry of microorganisms into the packaging in accordance with one or more embodiments of the presently claimed invention.
Figure 8 illustrates packaging implemented with a pouch and a tray in accordance with one or more examples.
Figure 9A illustrates a perspective of the cross-sectional views for Figures 9B-1 and 9B-2 in accordance with one or more examples.
Figure 9B-1 illustrates a cross-sectional view of an implementation of the packaging from Figure 9A where multiple packaging members/membranes are fused/bonded together to form the seal in accordance with one or more examples.

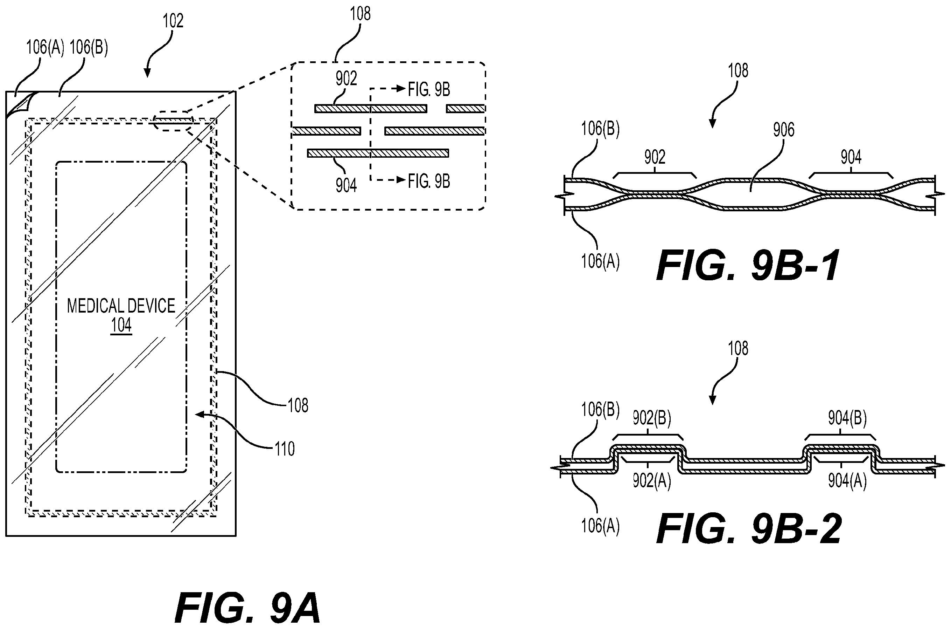

Figure 9B-2 illustrates a cross-sectional view of an implementation of the packaging from Figure 9A where multiple packaging members/membranes are coupled/attached together with mating features to form the seal in accordance with one or more examples.

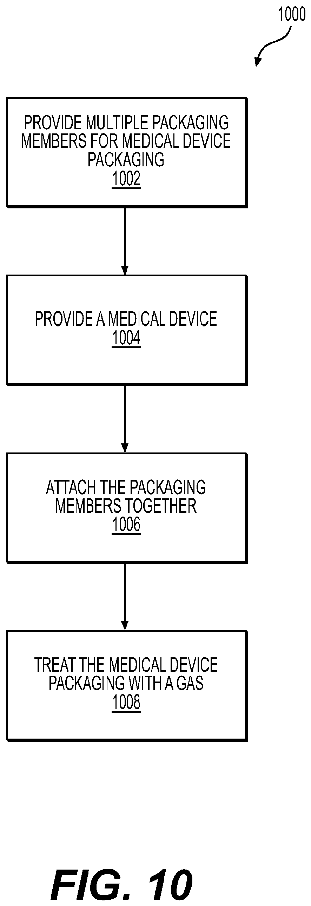

Figure 10 illustrates a flow diagram of a process for forming a gas-permeable channel in medical device packaging and treating the medical device packaging with a gas in accordance with one or more examples.

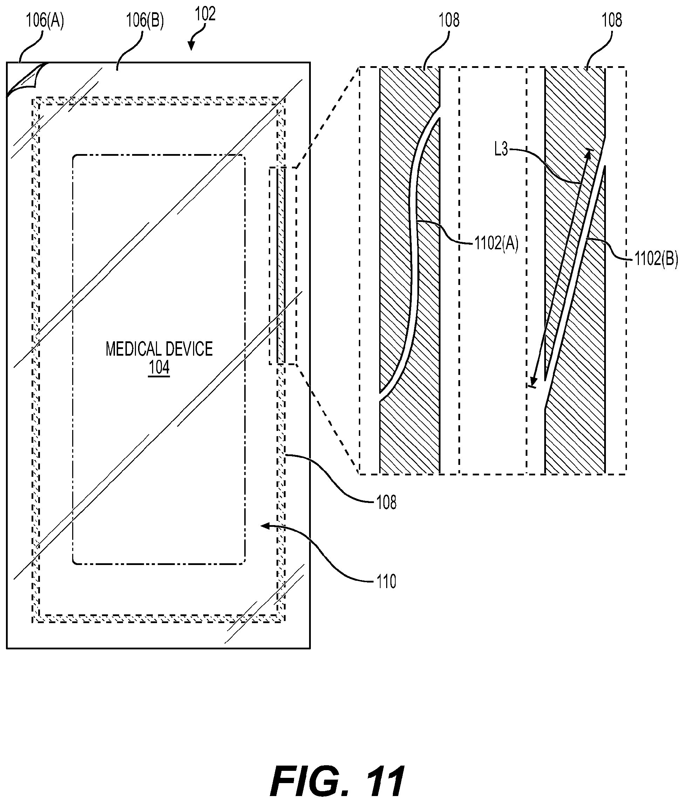

Figure 11 illustrates packaging that includes a single-track seal with a non-tortuous channel in accordance with one or more examples.

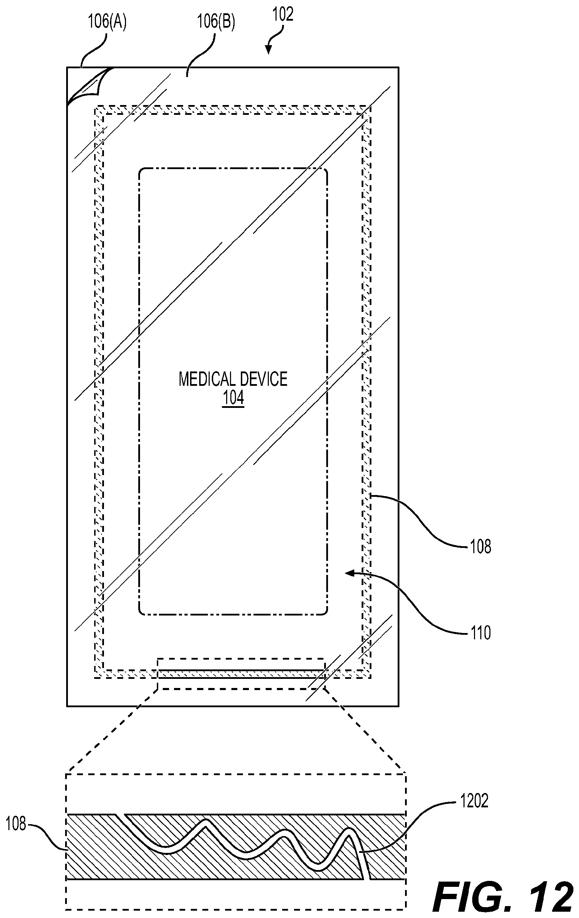

Figure 12 illustrates packaging that includes a single-track seal with a tortuous channel in accordance with one or more examples.

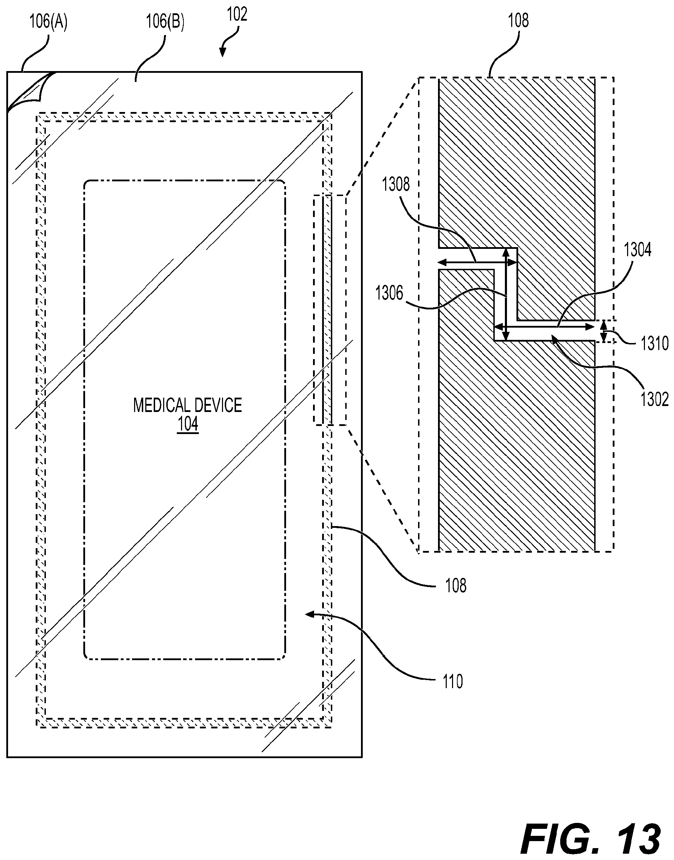

Figure 13 illustrates various dimensions of a channel formed in the seal of packaging in accordance with one or more examples.

### DETAILED DESCRIPTION

The headings provided herein are for convenience and do not necessarily affect the scope or meaning of the subject matter.

Although certain examples are disclosed below, the subject matter extends beyond the specifically disclosed examples to other alternative examples and/or uses and to modifications thereof. Thus, the scope of the claims that can arise here from is not limited by any of the examples described below. In any method or process disclosed herein, the acts or operations of the method or process can be performed in any suitable sequence and are not necessarily limited to any particular sequence. Various operations can be described as multiple discrete operations in turn, in a manner that can be helpful in understanding certain examples; however, the order of description should not be construed to imply that these operations are order dependent. Additionally, the structures, systems, and/or devices described herein can be embodied as integrated components or as separate components. For purposes of comparing various examples, certain aspects of these examples are described. Not necessarily all such aspects or advantages are achieved by any particular example. Thus, for example, various examples can be carried out in a manner that achieves or optimizes one advantage or group of advantages as taught herein without necessarily achieving other aspects or advantages as can also be taught or suggested herein.

Certain reference numbers are re-used across different figures of the figure set of the present disclosure as a matter of convenience for devices, components, systems, features, and/or modules having features that can be similar in one or more respects. However, with respect to any of the examples disclosed herein, re-use of common reference numbers in the drawings does not necessarily indicate that such features, devices, components, or modules are identical or similar. Rather, one having ordinary skill in the art can be informed by context with respect to the degree to which usage of common reference numbers can imply similarity between referenced subject matter. Use of a particular reference number in the context of the description of a particular figure can relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

Where an alphanumeric reference identifier is used that comprises a numeric portion and an alphabetic portion (e.g., '10a,' '10' is the numeric portion and 'a' is the alphabetic portion), references in the written description to the numeric portion (e.g., '10') can refer to any feature identified in the figures using such numeric portion (e.g., '10a,' '10b,' '10c,' etc.), even where such features are identified with reference identifiers that concatenate the numeric portion thereof with one or more alphabetic characters (e.g., 'a,' 'b,' 'c,' etc.). That is, a reference in the present disclosure to a feature '10' can be refer to either an identified feature '10a' in a particular figure of the present disclosure or to an identifier '10' or '10b' in the same figure or another figure, as an example.

Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, these terms are used herein for ease of description to describe the positional relationship between element(s)/structures(s), as illustrated in the drawings. Spatially relative terms are generally intended to encompass different orientations of the element(s)/structures(s), in use or operation, in addition to the orientations depicted in the drawings. For example, an element/structure described as "above" another element/structure can represent a position that is below or beside such other element/structure with respect to alternate orientations of the subject patient or element/structure, and vice-versa. Spatially relative terms, including those listed above, can be relative to a respective illustrated orientation of a referenced figure.

The present disclosure relates to devices, methods, and/or systems for packaging medical devices in a manner that facilitates sterilization or other processes of the medical devices disposed within the packaging and prohibits microbial ingress/penetration. For example, medical device packaging can include a gas-permeable channel/path that is configured to permit a gas to enter/exit the packaging where a medical device located while preventing/minimizing microbes from entering/exiting the packaging. In implementations, the medical device is disposed in the packaging for protection and/or distribution and subjected to a gas-sterilization process to sterilize or otherwise prepare the medical device. The gas-permeable channel can be implemented within/as part of a seal of the packaging, a surface of the packaging, and/or elsewhere. The gas-permeable channel can be a tortuous or non-tortuous path that inhibits/prevents microorganisms or other pathogens from contaminating the medical device. Further, in implementations, the gas-permeable channel includes a path to prevent microorganism entry and provide venting for the packaging (e.g., air venting from the inside of the packaging to the outside and/or from the outside to the inside) or other functions. Here, the packaging can be subjected to gas-sterilization and/or another form of sterilization, such as irradiation (where the seal may not be used for gas-sterilization entry). In implementations, the packaging includes multiple pieces that are formed of a same material. This can provide various sustainability benefits, such as allowing the packaging to be recycled or disposed of without fully separating the pieces of the packaging. Further, in implementations, the packaging includes one or more transparent portions to enable the medical device to be identified/examined from outside the packaging.

Any of the example methods and/or structures disclosed herein for treating a patient also encompass analogous methods and/or structures performed on or placed on a simulated patient, which is useful, for example, for training; for demonstration; for procedure and/or device development; and the like. The simulated patient can be physical, virtual, or a combination of physical and virtual. A simulation can include a simulation of all or a portion of a patient, for example, an entire body, a portion of a body (e.g., thorax), a system (e.g., cardiovascular system), an organ (e.g., heart), or any combination thereof. Physical elements can be natural, including human or animal cadavers, or portions thereof; synthetic; or any combination of natural and synthetic. Virtual elements can be entirely in silica, or overlaid on one or more of the physical components. Virtual elements can be presented on any combination of screens, headsets, holographically, projected, loud speakers, headphones, pressure transducers, temperature transducers, or using any combination of suitable technologies.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.).

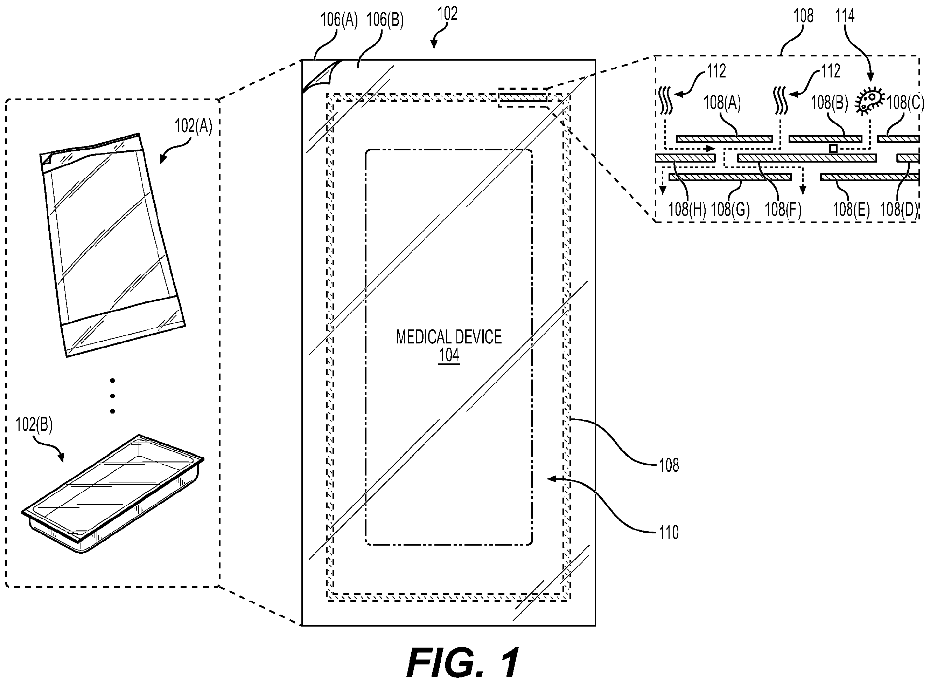

Figure 1 illustrates packaging/container/package 102 (also referred to as "the medical device packaging 102") that is configured to facilitate gas sterilization or another gaseous process to treat the packaging 102 and/or a medical device 104 disposed therein in accordance with one or more examples. In this implementation, the packaging 102 includes multiple packaging members/portions/sections 106 that are coupled to each, such as via a seal(s) 108. The packaging members 106 are sealed to form a cavity/area 110 within the packaging 102 to house/contain the medical device 104. The packaging members 106 can be separate elements/components or integral elements/components (e.g., a same material/substance including multiple portions/segments). The packaging members 106 can be sheets/layers/pieces/membranes of material, substances, etc. For ease of illustration/discussion, the packaging 102 is illustrated in various figures with the medical device 104 disposed therein; however, the packaging 102 is implemented without the medical device 104 in many examples. Furthermore, the packaging 102 can be configured to house other types of devices/items. In Figure 1, the callout to the right illustrates a top cross-sectional view of the seal 108.

In examples, the seal 108 includes one or more segments/portions positioned/formed to provide one or more channels (also referred to as "one or more pathways or paths") that facilitate passage through the seal 108. Figure 1 illustrates example seal segments 108(A)-108(H). Each seal segment can be a portion/section/area of the seal 108 where the packaging members 106(A) and 106(B) are attached/coupled together (e.g., in a sealed manner that prevents passage of a gas/vapor). Whereas an opening/discontinuity in the seal 108 can be a portion/section/area where the packaging members 106(A) and 106(B) are not attached/coupled together or attached together in a manner that permits passage of a gas/vapor. The collection of openings/discontinuities and/or spacing between one or more seal segments can form the one or more channels in the seal 108 (sometimes referred to as "one or more ingress pathways"). A channel and/or seal segment can include one or more straight and/or one or more curved portions/sections. A dimension (e.g., width, depth, length, etc.) of an opening or segment can be the same or different relative to another opening or segment.

In examples, the seal 108 includes one or more channels that form one or more tortuous paths, which can be configured to prevent microorganisms or other pathogens from reaching the medical device 104, since the microorganisms are unable to navigate a tortuous path. Further, in examples, the seal 108 includes one or more channels that include a particular length or other dimension such that an average distance or shortest/longest distance that is traveled from an external environment to the internal cavity 110 is more/less than a threshold. For example, a network of channels or single channel can be formed in the seal 108 so that a gas is required to travel at least a predetermined/threshold distance to reach the cavity 110 of the packaging 102 (e.g., a tortuous or non-tortuous path is longer than a threshold). This can prevent a pathogen from reaching the medical device 104, such as in cases where the microorganism may die after traveling a particular distance that is less than the predetermined/threshold distance.

In examples, such as that shown in Figure 1, the seal 108 includes multiple tracks that are separated from each other by a discontinuity/opening/spacing. The term "track" can represent a row, column, layer, level, trace, ring, etc. (often referred to as a "row" or "track" for convenience). Each track can include one or more segments disposed within a closed geometric shape/form. A track may take a variety of shapes/forms, such as when viewed from a top view like Figure 1 or another view, which may include a rectangle (as shown in Figure 1), circle, quadrilateral, line, etc. A track can be referenced/defined relative to a proximity/distance/shape of the track to the cavity 110/medical device 104, outer edge of the packaging 102, another feature of the packaging 102/medical device 104, etc. Figures 1-5 illustrate examples of the packaging 102 with the seal 108 having multiple tracks (also referred to as a "multi-track configuration").

To illustrate, in the example of Figure 1, the seal 108 includes three tracks that extend around the packaging 102 in horizontal direction and vertical directions, wherein each track is separated by other tracks by a distance (which can be the same distance or different relative to other tracks). In particular, seal segments 108(A), 108(B), and 108(C) form a first track (e.g., the segments 108(A), 108(B), and 108(C) are disposed within and/or make the shape of a first rectangular shape/track), the seal segments 108(H), 108(F), and 108(D) form a second track (e.g., the segments 108(H), 108(F), and 108(D) are disposed within and/or make the shape of a second rectangular shape/track), and the seal segments 108(G) and 108(E) form a third track (e.g., the segments 108(G) and 108(E) are disposed within and/or make the shape of a third rectangular shape/track). In the example of Figure 1, the tracks of the seal 108 generally include the same shape as the outer edge of the packaging 102. However, the tracks of the seal 108 can follow other features and/or form other shapes, which may or may not follow a feature of the packaging 102. In some instances, a track may be referred to as a row or column for ease of discussion, even if the track extends horizontal and/or vertical. For example, the outer most segments of the seal 108 of Figure 1 that extend around the packaging 102 can be part of the same row, even though the row extends horizontally and vertically with reference to the orientation of the packaging 102 shown in Figure 1.

Further, in examples, the seal 108 includes a single row/column/layer/level/ring/track with one or more channels formed therein. For instance, the seal 108 can include one or more segments disposed within a single closed geometric shape/form, such as that shown in Figures 11 and 12 discussed in further detail below.

In some implementations, one or more segments/portions of the seal 108 and/or one or more openings can be formed/disposed in a pattern to define predetermined/repetitive patterns for one or more channels/segments (also referred to as "a patterned seal"). For example, one or more first segments can include first dimensions and/or be positioned at first distances relative to each other and one or more second segments can include the first dimensions and/or be positioned at the first distances relative to each other. Further, in some implementations, one or more segments of the seal 108 and/or one or more openings can be formed/disposed in a random configuration to define random/non-repetitive channels/segments.

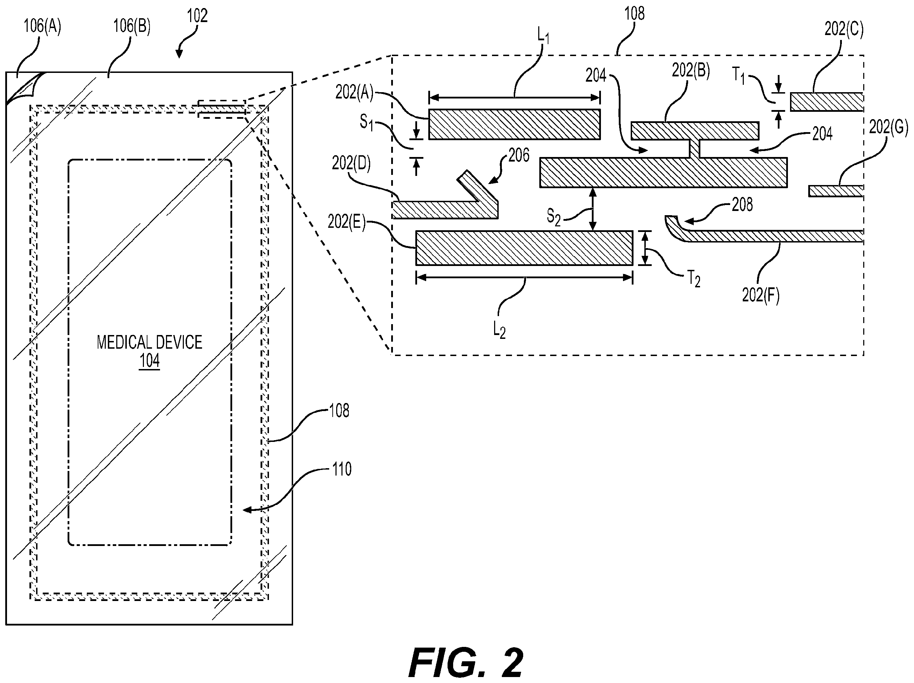

The seal 108 can include any number of entry channels/points into the seal 108 and/or any number of exit channels/points into the cavity 110. In some implementations, such as that illustrated in Figure 1, the seal 108 includes multiple entry channels/points to enter the seal 108 from the external environment and multiple exit channels/points for exiting from the seal 108 into the cavity 110. Further, in some implementations, the seal 108 includes a single-entry channel/point into the seal 108 and multiple exit channels/points into the cavity 110. The entry channel can diverge into multiple exit channels. Moreover, in some implementations, the seal 108 includes multiple entry channels/points into the seal 108 and a single exit channel/point. The entry channels can converge to the single exit channel. Furthermore, in some implementations, the seal 108 includes a single-entry channel/point and a single exit channel/point. As such, the one or more seal portions/segments of the seal 108 can take a variety of forms and/or positions to form a single path to the cavity 110 or multiple paths to the cavity 110. In some implementations, the one or more channels include one or more dead ends, as shown and discussed below in Figure 2.

The one or more seal segments/portions and/or one or more openings in the seal 108 can be sized/positioned to create specific characteristics for the one or more channels of the seal 108. In the example implementation of Figure 1, the seal 108 includes segments that have substantially the same thickness and/or spacing relative to each other, creating one or more channels that have substantially the same width. However, the seal 108 can include segments/openings/channels that have different characteristics, such as different lengths, thickness, widths, heights (e.g., distance between the packaging members 106(A) and 106(B)), spacing relative to each other (e.g., the vertical direction relative to Figure 1), spacing relative to an edge of the packaging 102, etc. For example, the seal 108 can include segments that have different thicknesses and/or have different spacing relative to each other.

In some implementations, the seal 108 is configured/designed such that an average distance or shortest/longest distance that is traveled from an external environment to the internal cavity 110 is more/less than a threshold. For example, a network of channels or single channel can be formed in the seal 108 so that a gas is required to travel at least a predetermined/threshold distance to reach the cavity 110 of the packaging 102 (e.g., a channel path is longer than a threshold). This can prevent a pathogen from reaching the medical device 104, such as in cases where the microorganism may die after traveling a particular distance that is less than the predetermined/threshold distance, the microorganism is unable to travel a particular distance along a path that includes a particular number of curves/corners/obstacles/features, the microorganism is unable to travel a particular distance for other reasons, etc. Further, in some implementations, the seal 108 is designed to include a number of curves/corners/obstacles/features, wherein such number can be more than (or less than) a threshold number associated with deterring/preventing microorganisms. Moreover, in some implementations, the seal 108 is designed to include one or more curves/corners/obstacles/features that have characteristics (e.g., angles, orientations, etc.) that are associated with deterring/preventing microorganisms.

The seal 108 can be formed in a variety of manners. In some implementations, the packaging 102 can be subjected to an energy source, such as heat, a laser, induction, etc., which emits energy/heat/beam to fuse/bond the packaging members 106(A) and 106(B) to each other to form the seal 108 and/or other attachment portions. Further, in some implementations, an adhesive is applied to the packaging members 106(A) and/or 106(B) to adhere/attach the packaging members 106(A) and 106(B) to each other to form the seal 108 and/or other attachment portions. Moreover, in some implementations, the packaging member 106(A) includes a mating feature configured to attach to an opposing matting feature on the packaging member 106(B), such as a flange, detent, indent, hole, protrusion, edge, etc. Here, the mating features can form the seal 108.

As shown in Figure 1, the seal 108 (and/or other portions of the packaging 102) includes one or more channels that are configured to permit a gas 112 to enter/exit the packaging 102 (e.g., the cavity 110) and/or prevent/minimize a pathogen 114 (e.g., microbes) from entering/exiting the packaging 102. The callout to the right in Figure 1 illustrates example paths that the gas 112 may take in reaching the medical device 104 housed within the packaging 102 and an example path that the pathogen 114 may take (e.g., before dying). The gas 112/pathogen 114 can navigate in a variety of manners along any number of pathways in an attempt to reach the cavity 110.

The packaging 102 can be implemented with a variety of materials. In some implementations, the packaging 102 includes multiple members/elements/portions that are each made of the same material (sometimes referred to as "mono-material packaging"). That is, the same type of material can be used for the members/elements/portions of the packaging 102. This can ease in recycling of the packaging 102 or otherwise disposing of the packaging 102 once the medical device 104 is removed. For example, the packaging members 106(A) and 106(B) can be made of the same material. In use, such as before/during a procedure, a user can partially or fully open the packaging 102, remove the medical device 104, and dispose of the packaging 102 into the same container. In some cases, by making packaging members 106(A) and 106(B) of the same material, the packaging 102 can be partially opened to remove the medical device 104 and the packaging 102 can be disposed of in the same recycling container (without the user having to fully separate the packaging members 106(A) and 106(B) from each other). This can minimize the effort of the user in removing the medical device 102, which can be beneficial in many instances, such as when relatively large packaging is used to house relatively large medical devices, when the user is required to remove multiple medical devices from packaging over the course of a day, etc. In other implementations, the packaging 102 includes different materials for multiple members/elements/portions of the packaging 102, such as a first material for the packaging member 106(A) and a second, different material for the packaging member 106(B).

In examples, the packaging 102 includes one or more members/elements/portions that are formed of a material that is substantially transparent/clear/see-through or non-transparent (opaque/translucent). In transparent implementations, a user can see into the cavity 110 of the packaging 102 to identify the medical device 104 disposed therein. The transparent/non-transparent material can include any type of polymeric material. For instance, the packaging members 106(A) and/or 106(B) can be transparent. In some implementations, the packaging 102 is formed of a single type of transparent material to enable the medical device 104 to be seen within the packaging 102 and to ease recycling of the packaging 102.

In examples, the packaging members 106(A) and/or 106(B) and/or other portions of the packaging 102 are implemented without a gas-permeable material. That is, the packaging members 106(A) and/or 106(B) and/or other portions of the packaging 102 can be implemented with non-porous material. For instance, the packaging 102 can be free of a gas-permeable sheet of material, such as Tyvek^{®}, medical paper, or another gas-permeable sheet. However, in other instances, the packaging 102 can include a gas-permeable sheet of material, in addition to, or separately from, the gas-permeable seal 108. A gas-permeable sheet can allow a gas to penetrate into the cavity 110 directly through the gas-permeable sheet.

As noted above, the packaging 102 can be subjected to a gas, such as the gas 112, to provide a treatment to the medical device 104 located within the packaging 102. For example, the medical device 104 can be disposed/placed within the packaging 102, the packaging 102 can be sealed with the seal 108, and then the packaging 102 can be subjected to the gas 112, which penetrates the packaging 102 through the seal 108 to enter the cavity 110 where the medical device 104 is located. However, the packaging 102 can be subjected to the gas 112 without the medical device 104 disposed therein, such as when the packaging 102 is being prepared to receive the medical device 104, the seal 108 is implemented as a resealable/reusable seal, the packaging 102 includes another way/means other than the seal 108 of enclosing the medical device 104 within the packaging, etc.

In some implementations, the packaging 102 is subjected to a gas, such as the gas 112, to perform a sterilization process, which sterilizes the medical device 104 disposed therein. A gas sterilization process can include treating the packaging 102 with Ethylene Oxide (ETO) gas, Vapor Hydrogen Peroxide, Nitrous Oxide, Chlorine Dioxide, Ozone (Gas Plasma), Steam sterilization, or another gas, while the medical device 104 is sealed within the packaging 102. The gas penetrates through the seal 108 (and/or other portions of the packaging 102, as discussed herein) to sterilize the medical device 102 and/or the packaging 102. In some implementations, a portion/member/membrane of the packaging 102 is non-porous for some substances and is breathable to gas, such as a polymeric material (e.g., Polytetrafluoroethylene). Although various examples are discussed in the context of using a gas to sterilize the medical device 104, a gas can be applied to treat the medical device 104 for other purposes.

Further, in some implementations, the packaging 102 is subjected to irradiation sterilization, including, but not limited to, E-Beam, Gamma, X-ray, etc.

The packaging 102 can be implemented in a variety of manners. In implementations, the packaging 102 is a pouch/bag/header bag/pouch-style packaging 102(A) that includes multiple sheets of material of relatively flexible material (e.g., flexible sheets) (also referred to as "a flexible package"). The multiple sheets of material can be subjected to an energy/heat source to seal the multiple sheets to each other and create a cavity for a medical device. Further, in implementations, the packaging 102 is a tray/jar/tray-style packaging 102(B) that includes a relatively rigid structure that may provide more protectioti/stippoil than a pouch (also referred to as "a rigid package"). Moreover, in implementations, the packaging 102 includes a sheet of a relatively flexible material (e.g., similar to or the same as a pouch sheet) that covers a tray. Further, the packaging 102 can include a tray located within a pouch or a pouch located within a tray.

Figure 2 illustrates details of the seal 108 that can be implemented in accordance with one or more examples. The seal 108 is illustrated with a plurality of segments/portions 202(A)-202(B), which can be similar to or the same as the one or more segments 108(A)-108(H) of Figure 1, although including the characteristics illustrated in Figure 2. As noted above, segments and/or channels formed by seal segments can have various characteristics depending on the configuration/design of the segments/channels, wherein one or more of the characteristics can be the same or different. Figure 2 illustrates various example characteristics that can be implemented in a variety of configurations of the seal 108. Although these characteristics are illustrated in a single example for ease of discussion, any of these characteristics can be implemented with or without the other characteristics of the segments 202. Figure 2 and other figures illustrate the seal 108 with a network of channels. In Figure 2, the callout to the right illustrates a top cross-sectional view of the seal 108.

For example, Figure 2 illustrates a thickness T₁ (i.e., a vertical distance of the segment 202(C) with respect to Figure 2). Here, the thickness T₁ of the segment 202(C) is smaller than a thickness T₂ of the segment 202(E). Further, Figure 2 illustrates a length L₁ of the segment 202(A) (i.e., horizontal distance of the segment 202(A) with respect to Figure 2), which is shorter than a length L₂ of the segment 202(E). Moreover, Figure 2 illustrates a spacing S₁ between the segments 202(A) and 202(B) (i.e., adjacent segments), which is smaller than a spacing S₂ between the segments 202(B) and 202(E).

Figure 2 illustrates that the segments 202 can include different shapes. For instance, the segment 202(B) includes multiple subsections that are joined together to define a non-rectangular shape, whereas the segment 202(A) includes a rectangular shape with respect to the cross-sectional view shown in Figure 2. Here, the segment 202(B) forms channels that include two dead ends 204. Further, the segment 202(D) includes a projection/angular section 206 that extends angularly off a main portion of the segment 202(D). Moreover, the segment 202(F) includes a curved section 208 that extends off an end of the segment 202(F). It should be understood that any of the segments 202 can include a curved section within any portion of the segment. A segment can be curved to form a curved channel. As such, the segments 202 can include a variety of shapes to facilitate a variety of forms/shapes of channels through the seal 108.

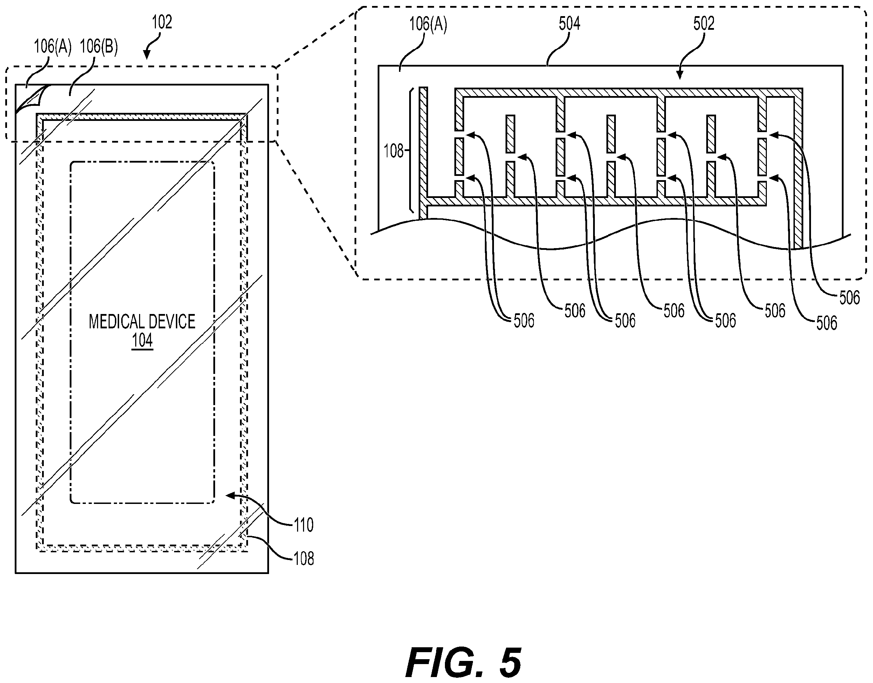

The segments 202 can be spaced/positioned such that some segments are located more distal to the cavity 110 (and more proximal to an external environment) and some segments are located more proximal to the cavity 110 (and more distal to the external environment). For example, the segment 202(A) is positioned closer/more proximal to the external environment and more distal to the cavity 110 than the segment 202(E). In other words, the segment 202(E) is positioned farther/more distal to the external environment and more proximal to the cavity 110 than the segment 202(A). As discussed herein, various segments of a seal can be represented/positioned in rows (such as that shown in Figure 1 or elsewhere), columns (such as that shown in Figure 5 or elsewhere), and/or in other configurations. A row can form a perimeter/periphery of the cavity 110.

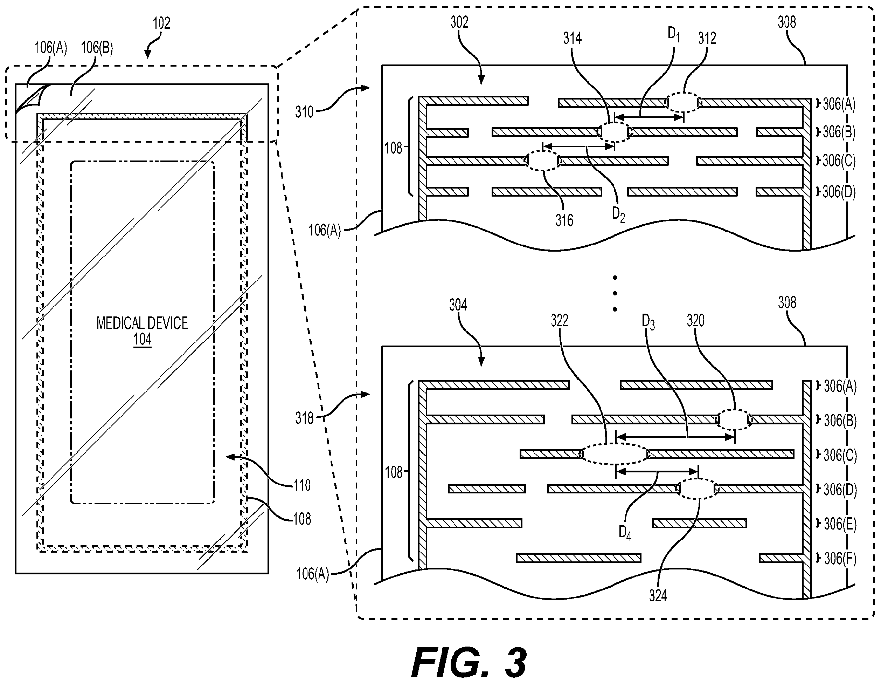

Figure 3 illustrates configurations/implementations of the seal 108 of the packaging 102 with multiple paths/channels through the seal 108 (similar to the configurations/implementations of Figures 1 and 2) in accordance with one or more examples. The seal 108 is illustrated with a plurality of segments/portions 302/304, which can be similar to or the same as the segments 108(A)-108(H) of Figure 1, although including the characteristics illustrated in Figure 3. In Figure 3, the callout to the right illustrates a top view of example configurations of the seal 108 with the top packaging member 106(B) removed and the seal 108 remaining (e.g., cross-sectional view through the seal 108).

In the implementations of Figure 3, the seal 108 includes rows 306 (also referred to as "tracks 306") that are equally spaced relative to each other. For instance, a distance/spacing/offset between the rows 306(A) and 306(B) (e.g., vertical distance relative to Figure 3) is the same as a distance between the rows 306(B) and 306(C), a distance between the rows 306(C) and 306(D) is the same as the distance between the rows 306(B) and 306(C), and so on. Here, the row 306(D) (e.g., segments of the row 306(D)) is positioned/located more proximal to the cavity 110 than the row 306(C) (e.g., segments of the row 306(C)), the row 306(C) is positioned/located more proximal to the cavity 110 than the row 306(B), and so on. However, a distance/spacing (whether vertical or horizontal) between any number of the rows 306 (or segments of the rows 306) can be different. In the example of Figure 3 with the segments 302, the seal 108 includes four rows 306 of seal segments, while the seal 108 includes six rows in the example with the segments 304. However, any number of rows can be implemented.

Further, in the implementations of Figure 3, the segments of the seal 108 are substantially elongate (also referred to as "elongate segments/sections") and oriented parallel to a particular edge of the packaging member 106(A), such as a top edge 308 that is closest to the seal 108 (e.g., closest to more than a threshold number of seal segments). This causes a gas/pathogen to travel in a substantially vertical/orthogonal/perpendicular direction (although it may also travel horizontally) relative to the top edge 308 to reach the cavity 110. In examples, the packaging 102 is configured to be opened from the top edge 308 (e.g., the packaging 102 includes tabs/peelable pieces at the top edge 308 that a user pulls to expose/remove the medical device 104). However, the seal segments can take other forms and/or be positioned in other manners, such as the configuration shown in Figure 5, discussed in further detail below.

At 310 (the top implementation shown in Figure 3), each of the rows 306 includes openings that are equally spaced relative to openings in an adjacent row. That is, each opening in a first row is horizontally offset with respect to an opening in the next/adjacent row by the same amount. For instance, a first opening 312 in the row 306(A) is located a distance from a second opening 314 in the row 306(B), illustrated with a horizontal distance D₁ between centers of the openings 312 and 314. Further, the second opening 314 in the row 306(B) is located a same distance from a third opening 316 in the row 306(C), illustrated with a horizontal distance D₂ between centers of the openings 314 and 316. That is, the distance D₁ is the same as the distance D₂. This can create a path/channel through the openings 312, 314, and 316. A distance between other openings in adjacent rows can be the same as well. In the implementation at 310, the segments of the seal 108 are patterned (e.g., distributed/positioned similarly/the same and/or sized similarly/the same) to create patterned channels.

At 318 (the bottom implementation shown in Figure 3), the rows 306 include at least some openings that are spaced differently relative to openings in an adjacent row. For instance, a first opening 320 in the row 306(B) is located a distance from a second opening 322 in the row 306(C), illustrated with a horizontal distance D₃ between centers of the openings 320 and 322. The second opening 322 in the row 306(C) is located a different distance from a third opening 324 in the row 306(D), illustrated with a horizontal distance D₄ between centers of the openings 314 and 316. That is, the distance D₃ is different than the distance D₄. A distance between other openings in adjacent rows can be the different as well (or the same). In the implementation at 318, the segments of the seal 108 are more randomly distributed (e.g., with less of a pattern) than the implementation at 310 to create random/non-patterned channels.

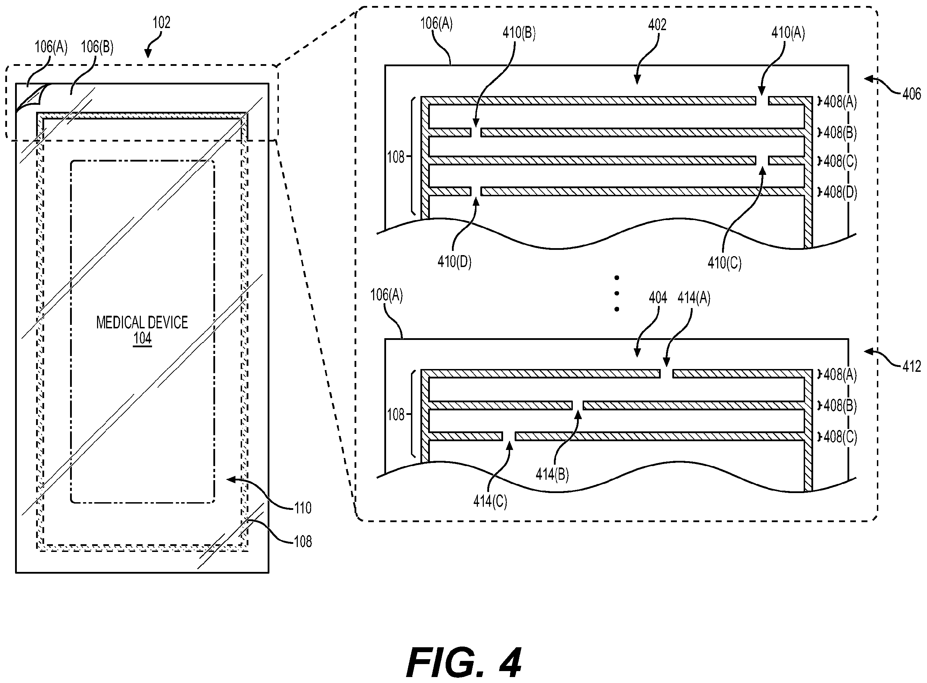

Figure 4 illustrates configurations/implementations of the seal 108 with a single path/channel through the seal 108 in accordance with one or more examples. The seal 108 is illustrated with a plurality of segments/portions 402/404, which can be similar to or the same as the segments 108(A)-108(H) of Figure 1, although including the characteristics illustrated in Figure 4. In Figure 4, the callout to the right illustrates a top view of example configurations of the seal 108 with the top packaging member 106(B) removed and the seal 108 remaining (e.g., cross-sectional view through the seal 108). In some examples, the configurations/implementations of Figure 4 are referred to as a labyrinth path. A labyrinth path can include a single opening (e.g., into the seal 108), a single exit (e.g., from the seal 108 into the cavity 110), and/or a repetitive pattern of seal segments/channels.

At 406 (the top implementation of Figure 4), the seal 108 is configured with openings 410 in the rows 408 that alternate from side to side. For example, the opening 410(A) is positioned on a first side of the packaging 102 (e.g., the right side), the opening 410(B) is positioned on a second side of the packaging 102 (e.g., the left side), the opening 410(C) is positioned on the first side of the packaging 102, and the opening 410(D) is positioned on the second side the packaging 102. In this example, the openings 410(A) and 410(C) are aligned vertically and the openings 410(B) and 410(D) are aligned vertically. However, the openings 410 may not be aligned vertically. Here, a channel is created that extends from right to left, down to the next channel through an opening in the seal 108, and then from left to right to the next opening in the seal 108. As such, an element can traverse the channel in a back-and-forth manner to reach the cavity 110. Each of the channels in between adjacent rows includes a dead end. However, the openings 410 can be positioned at the ends of the rows 406 to avoid dead ends. In this implementation, a distance between an opening on the right (i.e., openings 410(A) and 410(C)) and an opening on the left (i.e., openings 410(B) and 410(D)) is the same for each adjacent row. For example, a distance/spacing (e.g., horizontal distance) between the openings 410(A) and 410(B) is the same as a distance/spacing between the openings 410(B) and 410(C). In examples, the path/channel (e.g., tortuous path) through the seal 108 is longer than a threshold.

At 412 (the bottom implementation of Figure 4), the seal 108 is configured with openings 414 that incrementally move from one side of the packaging 102 to the other. For example, the opening 414(A) is positioned towards the right-side of the packaging 102, the opening 414(B) is positioned more towards the left-side of the packaging 102 relative to the opening 414(A) in the adjacent/above row, and the opening 414(C) is positioned even more towards the left-side of the packaging 102 relative to the opening 414(B) in the adjacent/above row. Here, a channel is created that extends from left-to-right and down through an opening, and then from left-to-right again and down through an opening. Although this example is illustrated in the context of channels/openings being formed from right-to-left, the alternative can be implemented (e.g., from left-to-right)

Figure 5 illustrates a configuration/implementation of the seal 108 with an at least partially parallel path/channel through the seal 108 with respect to an edge of the packaging 102 in accordance with one or more examples. The seal 108 is illustrated with a plurality of segments/portions 502, which can be similar to or the same as the segments 108(A)-108(H) of Figure 1, although including the characteristics illustrated in Figure 5. In Figure 5, the callout to the right illustrates a top view of an example configuration of the seal 108 with the top packaging member 106(B) removed and the seal 108 remaining (e.g., cross-sectional view through the seal 108).

In the implementation of Figure 5, the segments 502 of the seal 108 are substantially elongate and oriented orthogonal/perpendicular to a particular edge of the packaging member 106(A), such as a top edge 504 that is closest to the seal 108 (e.g., closest to more than a threshold number of seal segments). The segments 502 include openings 506 to provide one or more pathways/channels to the cavity 110. Here, an element, such as gas or pathogen, can enter the one or more channels at a first side of the packaging 102 (e.g., left side), traverse the seal 108 from left-to-right through one or more seal segments 502 and opening 506, and exit the seal 108 (enter the cavity 110) at the right side of the packaging 102. Although a single entry and single exit are illustrated, any number of entries/exits can be provided.

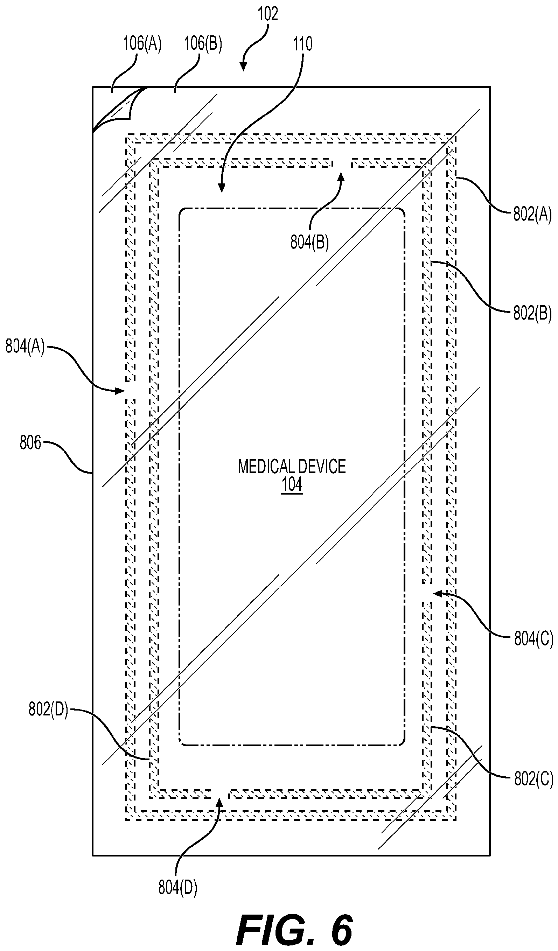

Figure 6 illustrates a configuration/implementation of a seal 802 for the packaging 102 that includes openings 804 positioned on different sides/edges of the packaging 102 in accordance with one or more examples. This provides a channel that extends from one side of the packaging 102 to another side of the packaging 102. The seal 802 is similar to or the same as the seal 108 of Figure 1, although including the characteristics illustrated in Figure 6.

As shown, the seal 802 includes the openings 804 to define/form seal segments 802(A)-802(D). In this implementation, the seal segment 802(A) (also referred to as "the outer track 802(A)") forms an outer segments/seal/perimeter (more distal to the cavity 110), while the seal segments 802(B)-802(D) (also referred to as "inner tracks 802(B)-802(D)") form inner segments/seals/perimeters (more proximal to the cavity 110). The inner segments 802(B)-802(D) can be positioned/spaced apart from the outer segment 802(A) to form a channel(s) therebetween. Although four seal segments are illustrated with three segments being inner seals, any number of seal segments can be implemented as inner or outer segments. In examples, the seal 802 can extend around a perimeter/edge(s) of the packaging 102.

As noted above, the openings 804 are positioned on different sides of the packaging 102 to facilitate channels that extend from one side to another side of the packaging 102. In this implementation, the opening 804(A) in the outer segment 802(A) is positioned on a first side of the packaging 102 (e.g., left side), while the openings 804(B)-804(D) in the inner segments 802(B)-802(D) are positioned on different sides of the packaging 102 (e.g., the top, right, and bottom). The openings can be positioned on adjacent or opposite sides of the packaging 102. For example, the opening 804(B) is positioned on an adjacent side relative to the opening 804(A). Meanwhile, the opening 804(C) is positioned on an opposite side relative to the opening 804(A). By positioning openings on other sides of the packaging 102, an element, such as a gas or pathogen, is forced to traverse from one side of the packaging 102 to another side of the packaging 102 to enter the cavity 110 where the medical device 102 is located.

In this implementation, the openings 804 are each offset from a center of the respective side on which the respective opening 804 is positioned. For example, the opening 802(A) is offset relative to a left side/edge 806 of the packaging 102. However, the openings 802 can be centered on the respective side or positioned in other locations along the respective side. Further, although a particular number of openings are illustrated, any number of openings 804 can be implemented.

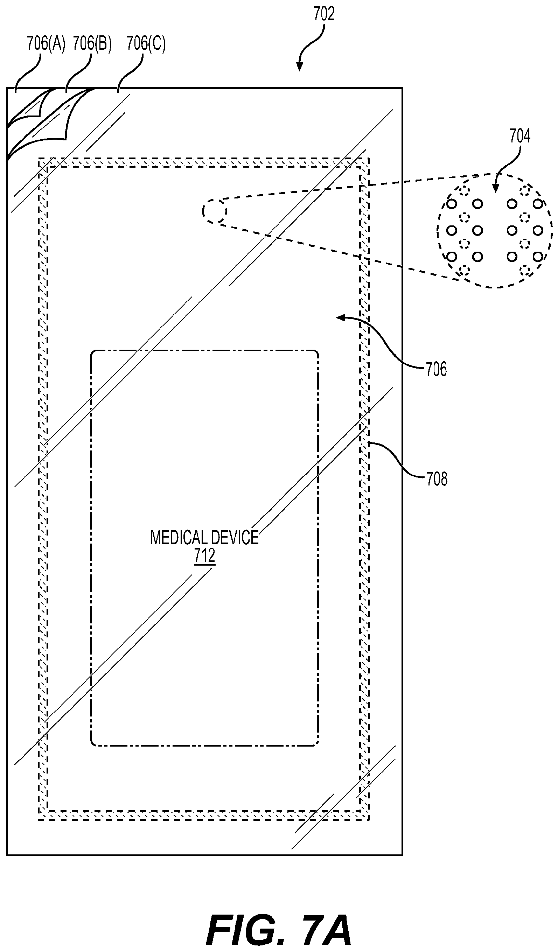

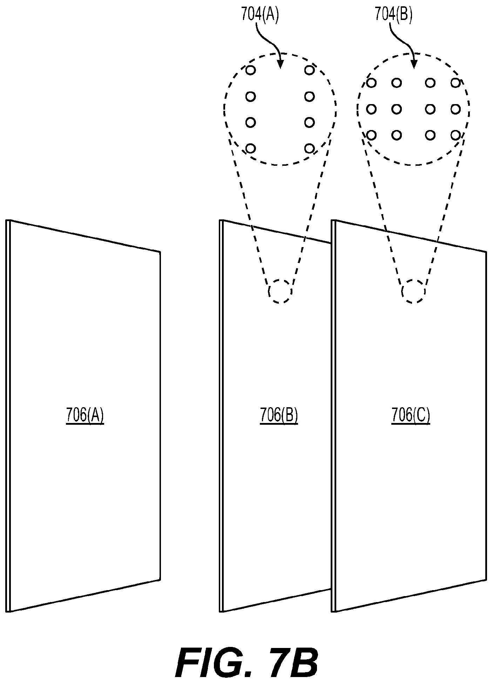

Figures 7A and 7B illustrate packaging 702 that includes pores/holes/perforations 704 configured in an offset manner on multiple packaging members/portions/sections 706 to facilitate a channel/path into the packaging 702 in accordance with one or more examples. Here, the packaging members 706 are implemented as sheets/layers/pieces/membranes of material; however, other forms can be implemented. The packaging 702 can be similar to or the same as the packaging 102, except that the packaging 702 includes pores 704 implemented on multiple packaging members 706. In some examples, the packaging 702 is referred to as "vented packaging."

As shown, the packaging 702 includes multiple packaging members 706(A)-706(C), which can be attached to each other around the perimeter/periphery of the packaging 102 and/or at other locations. In this example, the packaging 702 includes three packaging members 706; however, any number of packaging members can be implemented. Here, the packaging members 706 can be attached/coupled together (e.g., laminated, adhered, bonded, mated, etc.), such as via a seal 708 (which can be a gas-permeable seal similar to or the same as the seal 108 or a full/complete/non-permeable seal). As such, in some implementations, the packaging 706 includes multiple locations/elements where gas can penetrate into the packaging. The packaging members 706 can be attached together such that a cavity 710 is formed between the packaging members 706(A) and 706(B), wherein the cavity 710 is configured to house/contain a medical device 712.

In this implementation, the packaging members 706(B) and 706(C) include holes 704 (e.g., apertures, openings, fenestrations, etc.) that are offset/misaligned to form one or more channels through the packaging members 706(B) and 706(C) into the cavity 110. For instance, the packaging member 706(B) can include holes 704(A) positioned/formed in a first configuration/orientation/pattern and/or the packaging member 706(C) can include holes 704(B) positioned/formed in a second configuration/orientation/pattern, which can be the same as or different than the first configuration/orientation/pattern, as shown in Figure 7B for example. The packaging members 706(B) and 706(C) can be aligned such that at least some of the holes 704(A) and 704(B) (or more than a threshold number of holes) are not aligned. That is, a center of a hole 704(A) on the packaging member 706(B) is generally not aligned with a center of a hole 704(B) on the packaging member 706(C). The packaging members 706(B) and 706(C) can be disposed adjacent to each other with some amount of spacing/channeling therebetween (e.g., positioned on substantially offset/parallel planes, such as when formed, manufactured, provided without a medical device therein, or at other times). Such configuration can provide a channel/pathway (e.g., tortuous path) through the packaging members 706(B) and 706(C) to permit a gas to enter through the holes 704(B) in the packaging member 706(C), pass through a channel/spacing between the packaging members 706(B) and 706(C), and enter the cavity 110 through the holes 704(A).

The holes 704 can be positioned across an entirety of the packaging 102 or a specific area/section. In implementations, the holes 704 are formed in an area of the packaging members 706(B) and/or 706(C) that is sealed to form the cavity 110 (e.g., within the seal 708). Further, in implementations, the holes 704 are formed in an area of the packaging members 706(B) and/or 706(C) that is above/adjacent to the medical device 712. In some cases, the packaging members 706(B) and 706(C) are sealed together between adjacent holes 704 such that a gas is permitted to travel around the seal segments.

One or more of the holes 704 can include a relatively small diameter/dimension to permit entry of a gas to enter and minimize/prevent entry of other substances, such as a pathogen, liquid, etc. For example, individual ones of the holes 704 can include a diameter that is smaller than a particular number of microns. In some instances, the holes 704 are referred to as "micro-holes." The holes 704 can be formed in a variety of manners, which can include forming the holes 704 while manufacturing the individual packaging members 706(B) and 706(C).

In some implementations, the packaging 702 includes two packaging members/sheets/membranes implemented on one side of the cavity 10 (e.g., an outer/external sheet and inner sheet on a top side) and two packaging members/sheets implemented on another side (e.g., an outer/external sheet and inner sheet on an opposing/bottom side of the cavity 10). Here, a hole(s) can be positioned in one side on an external packaging sheet and a hole(s) can be positioned in an internal sheet on the other side of the packaging 702. A channel between the packaging sheets can be provided/formed/disposed around the cavity 110, such that a gas can travel through an external hole on one side of the packaging, through a channel between the packaging sheets, and through a hole on an internal sheet of the opposing side of the packaging 702 to reach the cavity 110.

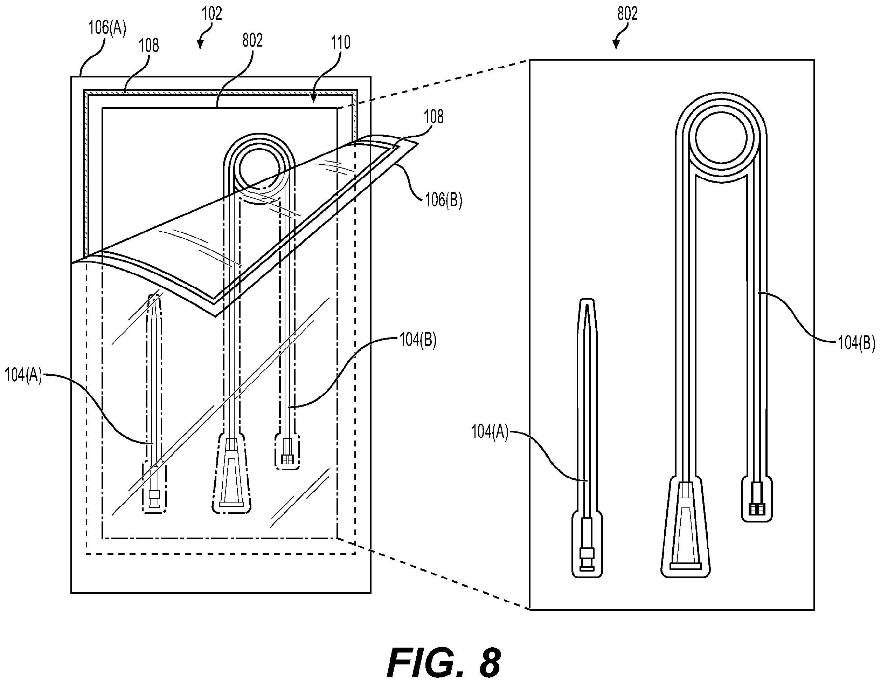

Figure 8 illustrates the packaging 102 implemented with a pouch and a tray 802 in accordance with one or more examples. Here, the pouch (also referred to as the pouch 106 in this example) is implemented with the packaging members 106(A) and 106(B), wherein the packaging members 106(A) and 106(B) are multiple sheets of material that are attached together at the seal 108. In this example, the top sheet 106(B) is partially peeled back to expose/reveal/access the medical device 104, which can include multiple components 104(A) and 104(B). An example access sheath 104(A) and catheter 104(B) are shown for illustrative purposes; however, any type of medical device or other device can be disposed therein.

The gas-permeable seal 108 can be implemented on the pouch 106 and/or the tray 802, such as to permit gas sterilization of the medical device 104. Here, the seal 108 is implemented between sheets of the pouch 106. Further, in implementations, a set of gas-permeable holes can be implemented on the pouch 106 and/or the tray 802.

In implementations, the pouch 106 is formed of a flexible material. Meanwhile, the tray 802 is formed of an at least partly rigid material (e.g., more rigid than the pouch 106), which can hold the medical device 104 and/or provide structure to protect the medical device 104. However, in some cases, the pouch 106 can be more rigid than the tray 802. The tray 802 can include grooves/depressions/cavities to receive/secure the medical device 104. In examples, the tray 802 includes multiple pieces that can attach together, such as a top tray/component and a bottom tray component that are configured with mating features to couple together around the medical device 104.

Figures 9A, 9B-1, and 9B-2 illustrate configurations/implementations of forming the seal 108 in accordance with one or more examples. For ease of discussion/illustration, the features of Figures 9A, 9B-1, and 9B-2 are discussed/illustrated in the context of the seal 108 of the packaging 102; however, such features can additionally, or alternatively, be implemented for the seal 708 of the packaging 702 and/or other seals discussed herein.

Figure 9B-1 illustrates a cross-sectional view of an implementation of the packaging 102 where the multiple packaging members 106(A) and 106(B) are fused/bonded together (e.g., laminated, melted, pressed, etc.) to form the seal 108 in accordance with one or more examples. For instance, an energy source (e.g., heat source, laser, etc.) can be configured to emit energy (e.g., heat, beam of light, etc.) in a specific location(s) to fuse/bond the packaging member 106(A) and 106(B) to each other to form seal segments 902 and 904 (e.g., gas-impermeable segments). Alternatively, or additionally, pressure can be applied in a specific location(s) to fuse/bond the packaging member 106(A) and 106(B) to each other to form seal segments 902 and 904. Areas where the packaging members 106(A) and 106(B) are not fused/bonded can form channels/spacing where a gas can pass through the seal 108, such as an area/spacing 906 illustrated in Figure 9B-1.

Figure 9B-2 illustrates a cross-sectional view of an implementation of the packaging 102 where the multiple packaging members 106(A) and 106(B) are coupled/attached together with mating features to form the seal 108 in accordance with one or more examples. For instance, the packaging member 106(A) can include a first mating feature 902(A) (e.g., male feature) configured to mate/couple to a second mating feature 902(B) (e.g., female feature) located on the packaging member 106(B) to form the seal segment 902. Further, the packaging member 106(A) can include a third mating feature 904(A) (e.g., male feature) configured to mate/couple to a fourth mating feature 904(B) located on the packaging member 106(B) to form the seal segment 904. A mating feature can include a flange, detent, indent, hole, protrusion, edge, etc.

In implementations, the packaging members 106(A) and 106(B) are bonded/fused together after being coupled via mating features. Moreover, in implementations (in the context of the features of either Figure 9B-1 or 9B-2), the packaging members 106(A) and 106(B) are attached/coupled together with an adhesive, such as at the seal segments 902 and 904.

Figure 10 illustrates a flow diagram of a process 1000 for forming a gas-permeable channel in medical device packaging and treating the medical device packaging with a gas in accordance with one or more examples. For ease of illustration, the process 1000 is discussed in the context of the example medical device packaging 102/702; however, the process 1000 can be implemented to form other medical device packaging.

At block 1002, the process 1000 includes providing multiple packaging members for medical device packaging. For example, one or more of the packaging members 106/706 can be provided. As noted herein, one or more of the packaging members 106/706 can be formed of the same material or different materials. In examples, one or more of the packaging members 106/706 includes a transparent material.

At block 1004, the process 1000 includes providing a medical device. For example, the medical device 104/712 can be provided for packaging within the medical device packaging 102/702.

At block 1006, the process 1000 includes attaching/coupling the multiple packaging members together. In one example, the packaging member 106(A) is attached/coupled to the packaging member 106(B) in a manner that forms the seal 108 (e.g., gas-permeable seal) between the members 106(A) and 106(B) and/or the cavity 110. This can include emitting energy with an energy source (e.g., in specific areas to form seal segments), pressing the packaging members 106(A) and 106(B) together (e.g., exerting force in specific areas to form seal segments), associating/coupling mating features, or otherwise performing a process to cotiple/attach the packaging members 106(A) and 106(B)- to form the seal 108.

In another example, the packaging members 706(A)-706(C) are attached/coupled together to provide a gas-permeable path between adjacent packaging members and/or provide a gas-permeable seal. For instance, the packaging members 706(B) and 706(C) can be positioned in a particular orientation/positioning and coupled together in the particular orientation/positioning such that the holes 704(A) and 704(B) are misaligned to provide a path through the multiple layers of the packaging members 706(B) and 706(C).

In implementations, the medical device 104/712 is disposed/positioned on or otherwise associated with one or more of the packaging members 106/706 such that the medical device 104/712 is sealed within the medical device packaging 102/702 as the packaging members 106/706 are coupled/attached together.

At block 1008, the process 1000 includes treating the medical device packaging with a gas. For example, the medical device packaging 102/702 can be treated with Ethylene Oxide (ETO) gas or another gas while the medical device 104/712 is disposed/seal within the medical device packaging 102/702. The gas can penetrate through the medical device packaging 102/702 and reach the medical device 104/712 through a gas-permeable channel(s), such as the gas-permeable seal 108, the holes 704, the seal 708, etc., wherein the gas-permeable channel(s) prevents a pathogen from reaching the medical device 104/712 inside the medical device packaging 102/702. As such, the gas-permeable channel(s) can provide a barrier from microbes and other pathogen.

Any of the various systems, devices, apparatuses, etc. in this disclosure can be sterilized (e.g., with heat, radiation, ethylene oxide, hydrogen peroxide, etc.) to ensure they are safe for use with patients, and/or the methods herein can comprise sterilization of the associated system, device, apparatus, etc. (e.g., with heat, radiation, ethylene oxide, hydrogen peroxide, etc.).

Figures 11 and 12 illustrate examples of the packaging 102 with the seal 108 implemented with a single track. For example, in contrast to other examples discussed herein, such as Figure 1 that includes multiple tracks for the seal 108, the packaging 102 of Figures 11 and 12 includes a single track for the seal 108 with one or more channels (also referred to as "one or more paths or pathways") to facilitate passage through the seal 108, such as to allow a gas/vapor to enter into the packaging 102 by passing through the single track. As discussed above, a channel can be an opening/discontinuity in the seal 108 where the packaging members 106(A) and 106(B) are not attached/coupled together or attached together in a manner that permits passage of a gas/vapor. As similarly discussed herein in the context of other examples, track of the seal 108 can include the same shape as the outer edge of the packaging 102, follow other features, or form other shapes, which may or may not be the same as a shape as a feature of the packaging 102. The example packaging 102 of Figures 11 and 12 can include any features discussed herein for other packaging.

Although the examples of Figures 11 and 12 illustrate a single channel through the seal 108 in the single track, the seal 108 can include multiple channels around the seal 108 that can each include an entry point and exit point. Further, in some single-track examples, the seal 108 includes multiple entry channels/points to enter the seal 108 from the external environment and multiple exit channels/points for exiting from the seal 108 into the cavity 110. Moreover, in some single-track examples, the seal 108 includes a single-entry channel/point into the seal 108 and multiple exit channels/points into the cavity 110. The entry channel can diverge into multiple exit channels. Moreover, in some single-track examples, the seal 108 includes multiple entry channels/points into the seal 108 and a single exit channel/point. The entry channels can converge to the single exit channel.

Figure 11 illustrates the packaging 102 with a non-tortuous channel(s) 1102 through the seal 108, wherein the non-tortuous channel 1102 is longer than a predetermined distance. The channel 1102 can be straight/substantially straight, curved, or take another form. In some examples, a non-tortuous channel includes less than a threshold number of turns/bends, less than a threshold number of curves, curves/bends by less than a particular amount/degrees, less than a threshold number of segments/sections between adjacent turns/obstacles/features, or other characteristics. The example non-tortuous channel 1102(A) illustrates a slightly curved path, while the example non-tortuous channel 1102(B) illustrates a straight path. Although the channel 1102(B) is referred to as a non-tortuous path, in some examples a slight curve in the channel (as shown) can be a tortuous path. A non-tortuous channel can include a length, width, or other dimension that is greater than a threshold/predetermined distance, such that a microorganism or gas would need to travel the length/dimension to reach the internal cavity 110. The threshold distance can be longer than a distance a microorganism can travel without being attached to a host/entity, which can be a distance that is based on experimental information indicating an average/longest/shortest distance a microorganism can travel without being attached to a host/entity. This can prevent the microorganism from reaching the medical device 104 within the packaging 102, such as in cases where the microorganism dies after traveling a particular distance that is less than the threshold distance. As shown in Figure 11, a length L₃ is representative of a length of the channel 1102, which can refer to a distance through the channel 1102 (e.g., a distance to travel through the channel 1102).

The non-tortuous channel 1102 can include a variety of forms and/or be disposed in a variety of locations. In examples, the non-tortuous channel 1102 can form a curved path on one side of the seal 108, such as that shown at 1102(A). Further, in examples, the non-tortuous channel 1102 can form a diagonal path within the seal 108, such as that shown at 1102(B). Moreover, in examples, the non-tortuous path 1102 can travel through the seal 108 from one side of the packaging 102 to another side of the packaging 102 through a corner/curve in the seal 108 (e.g., from the right side of the seal 108 to the top of the seal 108). Furthermore, in examples, the non-tortuous channel 1102 can be oriented relatively perpendicular to the seal 108 (e.g., the channel 102 can be perpendicular to a longitudinal axis of the seal 108).

In examples, an entry point and/or exit point of the non-tortuous channel 1102 can be positioned relative to a feature of the packaging 102 and/or a feature of the medical device 104 disposed therein. A feature can include any attribute or property of the packaging 102/medical device 104, such as a corner, an edge/side, a distal/proximal end, etc. For instance, an entry point (e.g., opening where a gas can enter the channel from the external environment) can be positioned at or within a predetermined proximity/distance to a first corner of the packaging 102 and an exit point (e.g., opening of the channel into the cavity 110) can be positioned at or within a predetermined proximity to a middle of the packaging 102, a second corner of the packaging 102, or another location on the packaging 102. Here, the channel 1102 can travel diagonally, straight, or in a curved manner.

In examples, the length L₃ of the channel 1102 is related/proportional to a dimension of the packaging 102, a dimension of the seal 108, a dimension of the medical device 104, etc. In one illustration, the length L₃ of the channel 1102 is greater than a predetermined threshold (e.g., the length L₃ is at least a minimum length), wherein the predetermined threshold is proportional to a dimension of the packaging 102/seal 108/medical device 104. Here, the length L₃ can be a quarter, third, half, three quarters, etc. of a length, width, or other dimension of the packaging 102/seal 108/medical device 104. In some examples, the length L₃ of the channel 1102 is based on a width of the channel, such as the length-width ratio or width-length ratio being a particular ratio, less than a threshold ratio, more than a threshold ratio, etc.

Figure 12 illustrates the packaging 102 with a tortuous channel(s) 1202 through the seal 108. A tortuous channel can include turns/bends or other features such that a path through the tortuous channel is not straight (e.g., indirect). In some examples, a tortuous channel includes more than a threshold number of turns, more than a threshold number of curves or other obstacles/features, curves/bends by more than a particular amount/degrees, more than a threshold number of segments/sections between adjacent turns/obstacles/features, or other characteristics. In some instances, a tortuous channel includes a particular pattern of features, while in other instances a tortuous channel includes features that are more randomly positioned/oriented along the path. A microorganism or other pathogen can generally not navigate/travel through a tortuous path, at least a tortuous path that includes sufficient turns, obstacles, or other features to navigate around. Thus, the packaging 102 can be implemented with the tortuous channel 1202 and/or the tortuous channel 1202 can be implemented with specific features to prevent microorganisms or other pathogens from reaching the medical device 104 disposed within the cavity 110. The tortuous channel 1202 can include the same or similar features as other tortuous channels discussed herein or elsewhere.

Further, in some examples, the tortuous channel 1202 is designed with a particular dimension to provide additional protection against microorganism entry. For instance, as similarly discussed above in reference to the packaging 102 shown in Figure 12, a length, width, or other dimension can be greater than a threshold/predetermined distance. The threshold distance can be longer than a distance a microorganism can travel without being attached to a host/entity, which can be a distance that is based on experimental information indicating an average/longest/shortest distance a microorganism can travel without being attached to a host/entity. This can prevent the microorganism from reaching the medical device 104 within the packaging 102, such as in cases where the microorganism dies after traveling a particular distance that is less than the threshold distance.

The tortuous channel 1202 can include a variety of forms and/or be disposed in a variety of locations. In examples, the tortuous path 1202 can travel through the seal 108 from one side of the packaging 102 to another side of the packaging 102 through a corner/curve in the seal 108 (e.g., from the right side of the seal 108 to the top of the seal 108). Further, in examples, the tortuous channel 1202 can be oriented relatively perpendicular to the seal 108 (e.g., a line between an entry point and exit point of the channel 1202 can be perpendicular to a longitudinal axis of the seal 108).

In examples, an entry point and/or exit point of the non-tortuous channel 1102 can be positioned relative to a feature of the packaging 102 and/or a feature of the medical device 104 disposed therein. As noted above, a feature can include any attribute or property of the packaging 102/medical device 104, such as a corner, an edge/side, a distal/proximal end, etc. For instance, an entry point of the tortuous channel 1102 (e.g., opening where a gas can enter the channel from the external environment) can be positioned at or within a predetermined proximity/distance to a first corner of the packaging 102 and an exit point of the tortuous channel 1102 (e.g., opening of the channel into the cavity 110) can be positioned at or within a predetermined proximity to a middle of the packaging 102, a second corner of the packaging 102, or another location on the packaging 102.

In examples, a length of the channel 1202 is related/proportional to a dimension of the packaging 102, a dimension of the seal 108, a dimension of the medical device 104, etc. In one illustration, the length of the channel 1202 is greater than a predetermined threshold (e.g., the length at least a minimum length), wherein the predetermined threshold is proportional to a dimension of the packaging 102/seal 108/medical device 104. Here, the length of the channel 1202 can be a quarter, third, half, three quarters, etc. of a length, width, or other dimension of the packaging 102/seal 108/medical device 104. In some examples, the length of the channel 1202 is based on a width of the channel, such as the length-width ratio or width-length ratio being a particular ratio, less than a threshold ratio, more than a threshold ratio, etc. Further, in some examples, the length of the channel 1202 is based on or otherwise related to a number of bends/turns in the channel 1202/seal 108. Similarly, a number of bends/turns in the channel 1202 can be based on or otherwise related to the length of the channel 1202/seal 108.

Figure 13 illustrates various dimensions of a channel 1302 formed in the seal 108 of the packaging 102. The channel 1302 can be representative of any of the channels discussed herein. In this example, the channel 1302 includes multiple sections/portions that include dimensions 1304, 1306, 1308, respectively. However, the channel 1302 can include a single section/portion and/or other characteristics, as discussed herein. For ease of discussion/illustration, each section is substantially straight. Each of the dimensions 1304, 1306, 1308 represents a length/distance of the respective section of the channel 1302 (e.g., a distance a microbe needs to travel through that section of the channel 1302) (sometimes referred to as "travel distance"). In examples, one or more of the dimensions 1304, 1306, and/or 1308 are at least .1 microns long (1 x 10⁻⁷ meters), 1 micron long, 2 microns long, 5 microns long, 10 microns long, 20 microns long, etc. In one illustration, each of the dimensions 1304, 1306, and 1308 is at least .1 microns long, micron long, 2 microns long, 5 microns long, 10 microns long, 20 microns long, etc. In another illustration, a total length of the channel 1302 (e.g., adding up all the dimensions 1304, 1306, and 1308) is at least .1 microns long, micron long, 2 microns long, 5 microns long, 10 microns long, 20 microns long, etc.

Figure 13 also shows a dimension 1310 of the channel 1302, which can represent a diameter, a largest cross-sectional dimension of the channel 1302, a smallest cross-sectional dimension of the channel 1302, a width of a cross-section of the channel 1302, a height of a cross-section of the channel 1302, an average cross-sectional dimension of the channel 1302, etc. The dimension 1310 can be uniform throughout a length of the channel 1310, such as from an opening to an exit, or nonuniform. In examples, the dimension 1310 can be at least .1 microns, .2 microns, .3 microns, .4 microns, etc. Further, in examples, the dimension 1310 (e.g., a diameter) can be in a range of .1 to .4 microns, a range of .1 to .3 microns, a range of .1 to .2 microns, etc. In examples, a cross-sectional area of the channel 1302 is at least 7.8 x 10⁻¹² square meters. In examples, a cross-sectional area of the channel 1302 is in a range of 7.8 x 10⁻¹² square meters to 1.26 x 10⁻¹¹ square meters (e.g., .1 microns diameter to .4 microns diameter) (e.g., for any of the lengths indicated above). In any of the above examples, the section of the channel 1302 can have a length that is at least 0.1 microns, at least 0.4 microns, in a range of .1 to .4 microns, in a range of .1 to .3 microns, or in a range of .1 to .2 microns. In any of the above examples, the section can have a total length that is less than 10 cm.

Although discussed in the context of the seal 108, the channel 1302 can be formed with multiple packaging members/portions/sections, such as in the example discussed for Figures 7A and 7B or elsewhere.

Depending on the example, certain acts, events, or functions of any of the processes or algorithms described herein can be performed in a different sequence, can be added, merged, and/or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements, and/or steps. Thus, such conditional language is not generally intended to imply that features, elements, and/or steps are in any way required for one or more examples or that one or more examples necessarily include logic for deciding, with or without author input or prompting, whether these features, elements, and/or steps are included or are to be performed in any particular example. The terms "comprising," "including," "having," and the like are generally synonymous, used in their ordinary sense, and used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y, and Z," unless specifically stated otherwise, is understood with the context as used in general to convey that an item, term, element, etc. can be either X, Y, or Z. Thus, such conjunctive language is not generally intended to imply that certain examples require at least one of X, at least one of Y, and at least one of Z to each be present.

In examples, various features are sometimes grouped together in a single example, figure, or description thereof for the purpose of streamlining the disclosure and aiding in the understanding of one or more of the various aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the subject matter herein disclosed and claimed below should not be limited by the particular examples described herein.

Certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather can generally distinguish the element from another element having a similar or identical name (but for use of the ordinal term). In addition, as used herein, indefinite articles ("a" and "an") can indicate "one or more" rather than "one." Further, an operation performed "based on" a condition or event can also be performed based on one or more other conditions or events not explicitly recited.

Unless otherwise defined, terms (including technical and/or scientific terms) used herein can have the same meaning as commonly understood by one of ordinary skill in the art to which examples belong. Terms, such as those defined in commonly used dictionaries, can be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, can be used herein for ease of description to describe the relations between one element or component and another element or component as illustrated in the drawings. Spatially relative terms can encompass different orientations of the device in use or operation, in addition to the orientation depicted in the drawings. For example, in the case where a device shown in the drawing is turned over, the device positioned "below" or "beneath" another device can be placed "above" another device. Accordingly, the illustrative term "below" can include both the lower and upper positions. The device can also be oriented in the other direction, and thus the spatially relative terms can be interpreted differently depending on the orientations.

Unless otherwise expressly stated, comparative and/or quantitative terms, such as "less," "more," "greater," and the like, can encompass the concepts of equality. For example, "less" can mean not only "less" in the strictest mathematical sense, but also "less than or equal to."

## Claims

1. Medical device packaging (702) comprising:
a first packaging member (706B); and
a second packaging member (706A) attached to the first packaging member with a gas-permeable seal (708), the first packaging member and the second packaging member forming an inner cavity (710) configure to house a medical device (712), the gas-permeable seal including a channel configured to permit gas passage through the gas-permeable seal and impede microorganism entry into the inner cavity, the channel including at least one of: 1) a tortuous path; or 2) a path that is longer than a predetermined threshold; and
at least one of the first packaging member or the second packaging member being substantially transparent;
wherein the first packaging member includes one or more first holes (704A), the medical device packaging further comprising:
a third packaging member (706C) disposed adjacent to the first packaging member, the third packaging member including one or more second holes (704B) offset from the one or more first holes, the first and third packaging members forming a tortuous channel to the inner cavity through the one or more first holes, a spacing between the first and third packaging members, and the one or more second holes.

2. The medical device packaging of claim 1, wherein the first and second packaging members are:
a) substantially transparent; and/or
b) formed of the same material.

3. The medical device packaging of any of claim 1 or claim 2, wherein the gas-permeable seal includes at least a first seal segment (108A) and a second seal segment (108E) spaced apart from the first seal segment, the second seal segment being located more proximal to the inner cavity than the first seal segment.

4. The medical device packaging of claim 3, wherein the first seal segment includes a first opening (312, 320) and the second seal segment includes a second opening (314, 322) located a first distance (D1, D3) from the first opening.

5. The medical device packaging of claim 4, wherein the gas-permeable seal includes a third seal segment that is spaced apart from the second seal segment, the third seal segment being located more proximal to the inner cavity than the second seal segment, the third seal segment including a third opening (316, 324) located a second distance (D2, D4) from the second opening.

6. The medical device packaging of claim 5, wherein a spacing (51) between the first seal segment (202A) and the second seal segment (202B) is:
a) different than a spacing (52) between the second seal segment (202B) and the third seal segment (202E); or
b) substantially the same as a spacing between the second seal segment and the third seal segment.

7. The medical device packaging of claim 5, wherein the first distance (D3) between the first opening (320) and the second opening (322) is:
a) different than the second distance (D4) between the second opening (322) and the third opening (324); or
b) substantially the same as the second distance (D2) between the second opening (314) and the third opening (316).

8. The medical device packaging of claim 4, wherein the first opening (410A) is located on a first side of the medical device packaging and the second opening (410B) is located on a second side of the medical device packaging.

9. The medical device packaging of claim 3, wherein the first seal segment (302, 502, 802B) is an:
a) elongate segment that is positioned substantially parallel to an edge (308) of the medical device packaging;
b) elongate segment that is positioned substantially orthogonal to an edge (502) of the medical device packaging; or
c) inner track around a perimeter of the medical device packaging and the second seal segment (802A) is an outer track around the perimeter.

10. The medical device packaging of any of claims 1-9, wherein:
a) the predetermined threshold of the path of the channel is at least x 10⁻⁷ meters in length; and/or
b) a cross-sectional area of the path of the channel is in a range of 7.8 x 10⁻¹² square meters to 1.26 x 10⁻¹¹ square meters, optionally wherein the path having the cross-sectional area in the range of 7.8 x 10⁻¹² square meters to 1.26 x 10⁻¹¹ square meters extends at least x 10⁻⁷ meters in length.

11. The medical device packaging of any of claims 1-10, wherein the first and second packaging members are each formed of a non-porous material.

12. The medical device packaging of any of claims 1-11, wherein the gas-permeable seal includes a first mating feature (902A) on the first packaging member and a second mating feature (902B) on the second packaging member that is configured to attach to the first mating feature.

13. The medical device packaging of any of claims 1-12, wherein the medical device packaging is:
a) a flexible package; or
b) a rigid package.

14. The medical device packaging of any of claims 1-13, wherein the gas-permeable seal:
a) is implemented as a single-track seal with one or more seal segments positioned on a single track; or
b) includes at least one seal segment (108D) implemented on a first track and at least one seal segment (108A) implemented on a second track, the first track being closer to the inner cavity than the second track.

15. The medical device packaging of any of claims 1-14, wherein the channel includes the path that is longer than the predetermined threshold:
a) the path being substantially straight;
b) the predetermined threshold being related to a dimension of the medical device packaging; and/or
c) the predetermined threshold being proportional to a length of the gas-permeable seal.

## Patentansprüche

1. Medizinische-Vorrichtung-Verpackung (702), die Folgendes umfasst:
ein erstes Verpackungselement (706B); und
ein zweites Verpackungselement (706A), das mit einer gasdurchlässigen Dichtung (708) an dem ersten Verpackungselement angebracht ist, wobei das erste Verpackungselement und das zweite Verpackungselement einen inneren Hohlraum (710) bilden, der dazu konfiguriert ist, eine medizinische Vorrichtung (712) zu beherbergen, wobei die gasdurchlässige Dichtung einen Kanal beinhaltet, der dazu konfiguriert ist, einen Gasdurchgang durch die gasdurchlässige Dichtung zu ermöglichen und das Eindringen von Mikroorganismus in den inneren Hohlraum zu behindern, wobei der Kanal wenigstens eines von Folgendem beinhaltet: 1) einen gewundenen Pfad; oder 2) einen Pfad, der länger als eine vorbestimmte Schwelle ist; und
wobei das erste Verpackungselement und/oder das zweite Verpackungselement im Wesentlichen transparent ist;
wobei das erste Verpackungselement ein oder mehrere erste Löcher (704A) beinhaltet, wobei die Medizinische-Vorrichtung-Verpackung ferner Folgendes umfasst:
ein drittes Verpackungselement (706C), das angrenzend an das erste Verpackungselement angeordnet ist, wobei das dritte Verpackungselement ein oder mehrere zweite Löcher (704B) beinhaltet, die von dem einen oder den mehreren ersten Löchern versetzt sind, wobei das erste und dritte Verpackungselement einen gewundenen Kanal zu dem inneren Hohlraum durch das eine oder die mehreren ersten Löcher, eine Beabstandung zwischen dem ersten und dritten Verpackungselement und das eine oder die mehreren zweiten Löchern bilden.

2. Medizinische-Vorrichtung-Verpackung nach Anspruch 1, wobei das erste und zweite Verpackungselement Folgendes sind:
a) im Wesentlichen transparent; und/oder
b) aus dem gleichen Material gebildet.

3. Medizinische-Vorrichtung-Verpackung nach einem der Ansprüche 1 oder 2, wobei die gasdurchlässige Dichtung wenigstens ein erstes Dichtungssegment (108A) und ein zweites Dichtungssegment (108E) beinhaltet, das von dem ersten Dichtungssegment beabstandet ist, wobei sich das zweite Dichtungssegment näher an dem inneren Hohlraum als das erste Dichtungssegment befindet.

4. Medizinische-Vorrichtung-Verpackung nach Anspruch 3, wobei das erste Dichtungssegment eine erste Öffnung (312, 320) beinhaltet und das zweite Dichtungssegment eine zweite Öffnung (314, 322) beinhaltet, die sich in einem ersten Abstand (D1, D3) von der ersten Öffnung befindet.

5. Medizinische-Vorrichtung-Verpackung nach Anspruch 4, wobei die gasdurchlässige Dichtung ein drittes Dichtungssegment beinhaltet, das von dem zweiten Dichtungssegment beabstandet ist, wobei sich das dritte Dichtungssegment näher an dem inneren Hohlraum als das zweite Dichtungssegment befindet, wobei das dritte Dichtungssegment eine dritte Öffnung (316, 324) beinhaltet, die sich in einem zweiten Abstand (D2, D4) von der zweiten Öffnung befindet.

6. Medizinische-Vorrichtung-Verpackung nach Anspruch 5, wobei eine Beabstandung (51) zwischen dem ersten Dichtungssegment (202A) und dem zweiten Dichtungssegment (202B) Folgendes ist:
a) verschieden von einer Beabstandung (52) zwischen dem zweiten Dichtungssegment (202B) und dem dritten Dichtungssegment (202E); oder
b) im Wesentlichen gleich einer Beabstandung zwischen dem zweiten Dichtungssegment und dem dritten Dichtungssegment.

7. Medizinische-Vorrichtung-Verpackung nach Anspruch 5, wobei der erste Abstand (D3) zwischen der ersten Öffnung (320) und der zweiten Öffnung (322) Folgendes ist:
a) verschieden von dem zweiten Abstand (D4) zwischen der zweiten Öffnung (322) und der dritten Öffnung (324); oder
b) im Wesentlichen gleich dem zweiten Abstand (D2) zwischen der zweiten Öffnung (314) und der dritten Öffnung (316).

8. Medizinische-Vorrichtung-Verpackung nach Anspruch 4, wobei sich die erste Öffnung (410A) auf einer ersten Seite der Medizinische-Vorrichtung-Verpackung befindet und sich die zweite Öffnung (410B) auf einer zweiten Seite der Medizinische-Vorrichtung-Verpackung befindet.

9. Medizinische-Vorrichtung-Verpackung nach Anspruch 3, wobei das erste Versiegelungssegment (302, 502, 802B) Folgendes ist:
a) ein längliches Segment, das im Wesentlichen parallel zu einem Rand (308) der Medizinische-Vorrichtung-Verpackung positioniert ist;
b) ein längliches Segment, das im Wesentlichen orthogonal zu einem Rand (502) der Medizinische-Vorrichtung-Verpackung positioniert ist; oder
c) eine innere Spur um einen Perimeter der Medizinische-Vorrichtung-Verpackung herum, und das zweite Dichtungssegment (802A) eine äußere Spur um den Perimeter herum ist.

10. Medizinische-Vorrichtung-Verpackung nach einem der Ansprüche 1-9, wobei:
a) die vorbestimmte Schwelle des Pfades des Kanals wenigstens 1 x 10⁻⁷ Meter lang ist; und/oder
b) eine Querschnittsfläche des Pfades des Kanals in einem Bereich von 7,8 x 10⁻¹² Quadratmeter bis 1,26 x 10⁻¹¹ Quadratmeter liegt, wobei sich optional der Pfad mit der Querschnittsfläche in dem Bereich von 7,8 x 10⁻¹² Quadratmeter bis 1,26 x 10⁻¹¹ Quadratmeter über eine Länge von wenigstens 1 x 10⁻⁷ Meter erstreckt.

11. Medizinische-Vorrichtung-Verpackung nach einem der Ansprüche 1-10, wobei das erste und zweite Verpackungselement jeweils aus einem nichtporösen Material gebildet sind.

12. Medizinische-Vorrichtung-Verpackung nach einem der Ansprüche 1-11, wobei die gasdurchlässige Dichtung ein erstes Passmerkmal (902A) an dem ersten Verpackungselement und ein zweites Passmerkmal (902B) an dem zweiten Verpackungselement beinhaltet, das dazu konfiguriert ist, an dem ersten Passmerkmal befestigt zu werden.

13. Medizinische-Vorrichtung-Verpackung nach einem der Ansprüche 1-12, wobei die Medizinische-Vorrichtung-Verpackung Folgendes ist:
a) eine flexible Verpackung; oder
b) eine starre Verpackung.

14. Medizinische-Vorrichtung-Verpackung nach einem der Ansprüche 1-13, wobei die gasdurchlässige Dichtung:
a) als einspurige Dichtung mit einem oder mehreren Dichtungssegmenten implementiert ist, die auf einer einzigen Spur positioniert sind; oder
b) wenigstens ein Dichtungssegment (108D), das auf einer ersten Spur implementiert ist, und mindestens ein Dichtungssegment (108A), das auf einer zweiten Spur implementiert ist, beinhaltet, wobei die erste Spur näher an dem inneren Hohlraum als die zweite Spur ist.

15. Medizinische-Vorrichtung-Verpackung nach einem der Ansprüche 1-14, wobei der Kanal den Pfad beinhaltet, der länger als die vorbestimmte Schwelle ist:
a) wobei der Pfad im Wesentlichen gerade ist;
b) wobei sich die vorbestimmte Schwelle auf eine Dimension der Medizinische-Vorrichtung-Verpackung bezieht; und/oder
c) wobei die vorbestimmte Schwelle proportional zu einer Länge der gasdurchlässigen Dichtung ist.

## Revendications

1. Emballage de dispositif médical (702) comprenant :
un premier élément d'emballage (706B) ; et
un deuxième élément d'emballage (706A) fixé au premier élément d'emballage avec un joint d'étanchéité perméable aux gaz (708), le premier élément d'emballage et le deuxième élément d'emballage formant une cavité interne (710) conçue pour accueillir un dispositif médical (712), le joint d'étanchéité perméable aux gaz comportant un canal conçu pour permettre le passage de gaz à travers le joint d'étanchéité perméable aux gaz et empêcher l'entrée de micro-organismes dans la cavité interne, le canal comprenant au moins un élément parmi : 1) un passage tortueux ; ou 2) un passage qui est plus long qu'un seuil prédéterminé ; et
le premier élément d'emballage et/ou le deuxième élément d'emballage étant sensiblement transparents ;
le premier élément d'emballage comportant un ou plusieurs premiers trous (704A), l'emballage de dispositif médical comprenant en outre :
un troisième élément d'emballage (706C) disposé au voisinage du premier élément d'emballage, le troisième élément d'emballage comportant un ou plusieurs deuxièmes trous (704B) décalés par rapport au(x) premier(s) trou(s), les premier et troisième éléments d'emballage formant un canal tortueux jusqu'à la cavité interne passant par le ou les premiers trous, un espace entre le premier et le troisième élément d'emballage, et le ou les deuxièmes trous.

2. Emballage de dispositif médical de la revendication 1, dans lequel les premier et deuxième éléments d'emballage sont :
a) sensiblement transparent ; et/ou
b) constitués du même matériau.

3. Emballage de dispositif médical de l'une quelconque des revendications 1 et 2, dans lequel le joint d'étanchéité perméable aux gaz comporte au moins un premier segment de joint d'étanchéité (108A) et un deuxième segment de joint d'étanchéité (108E) espacé du premier segment de joint d'étanchéité, le deuxième segment de joint d'étanchéité étant situé plus près de la cavité interne que le premier segment de joint d'étanchéité.

4. Emballage de dispositif médical de la revendication 3, dans lequel le premier segment de joint d'étanchéité comporte une première ouverture (312, 320) et le deuxième segment de joint d'étanchéité comporte une deuxième ouverture (314, 322) située à une première distance (D1, D3) de la première ouverture.

5. Emballage de dispositif médical de la revendication 4, dans lequel le joint d'étanchéité perméable aux gaz comporte un troisième segment de joint d'étanchéité qui est espacé du deuxième segment de joint d'étanchéité, le troisième segment de joint d'étanchéité étant situé plus près de la cavité interne que le deuxième segment de joint d'étanchéité, le troisième segment de joint d'étanchéité comportant une troisième ouverture (316, 324) située à une deuxième distance (D2, D4) de la deuxième ouverture.

6. Emballage de dispositif médical de la revendication 5, dans lequel un espacement (51) entre le premier segment de joint d'étanchéité (202A) et le deuxième segment de joint d'étanchéité (202B) est :
a) différent d'un espacement (52) entre le deuxième segment de joint d'étanchéité (202B) et le troisième segment de joint d'étanchéité (202E) ; ou
b) sensiblement égal à un espacement entre le deuxième segment de joint d'étanchéité et le troisième segment de joint d'étanchéité.

7. Emballage de dispositif médical de la revendication 5, dans lequel la première distance (D3) entre la première ouverture (320) et la deuxième ouverture (322) est :
a) différente de la deuxième distance (D4) entre la deuxième ouverture (322) et la troisième ouverture (324) ; ou
b) sensiblement égale à la deuxième distance (D2) entre la deuxième ouverture (314) et la troisième ouverture (316).

8. Emballage de dispositif médical de la revendication 4, dans lequel la première ouverture (410A) se situe sur un premier côté de l'emballage de dispositif médical et la deuxième ouverture (410B) se situe sur un deuxième côté de l'emballage de dispositif médical.

9. Emballage de dispositif médical de la revendication 3, dans lequel le premier segment de joint d'étanchéité (302, 502, 802B) est :
a) un segment allongé qui est positionné de façon sensiblement parallèle à un bord (308) de l'emballage de dispositif médical ;
b) un segment allongé qui est positionné de façon sensiblement orthogonale à un bord (502) de l'emballage de dispositif médical ; ou
c) une bande interne autour d'un périmètre de l'emballage de dispositif médical, et le deuxième segment de joint d'étanchéité (802A) est une bande externe autour du périmètre.

10. Emballage de dispositif médical de l'une quelconque des revendications 1 à 9, dans lequel :
a) le seuil prédéterminé du passage du canal est d'au moins 1 x 10⁻⁷ mètre en longueur ; et/ou
b) une aire de section transversale du passage du canal est comprise entre 7,8 x 10⁻¹² mètre carré et 1,26 x 10⁻¹¹ mètre carré, le passage ayant l'aire de section transversale comprise entre 7,8 x 10⁻¹² mètre carré et 1,26 x 10⁻¹¹ mètre carré s'étendant éventuellement sur au moins 1 x 10⁻⁷ mètre en longueur.

11. Emballage de dispositif médical de l'une quelconque des revendications 1 à 10, dans lequel les premier et deuxième éléments d'emballage sont chacun constitués d'un matériau non poreux.

12. Emballage de dispositif médical de l'une quelconque des revendications 1 à 11, dans lequel le joint d'étanchéité perméable aux gaz comporte un premier élément d'emboîtement (902A) sur le premier élément d'emballage et un deuxième élément d'emboîtement (902B) sur le deuxième élément d'emballage qui est conçu pour se fixer au premier élément d'emboîtement.

13. Emballage de dispositif médical de l'une quelconque des revendications 1 à 12, l'emballage de dispositif médical étant :
a) un emballage souple ; ou
b) un emballage rigide.

14. Emballage de dispositif médical de l'une quelconque des revendications 1 à 13, dans lequel le joint d'étanchéité perméable aux gaz :
a) est mis en œuvre sous la forme d'un joint d'étanchéité à une seule bande avec un ou plusieurs segments de joint d'étanchéité positionnés sur une seule bande ; ou
b) comporte au moins un segment de joint d'étanchéité (108D) mis en œuvre sur une première bande et au moins un segment de joint d'étanchéité (108A) mis en œuvre sur une deuxième bande, la première bande étant plus proche de la cavité interne que la deuxième bande.

15. Emballage de dispositif médical de l'une quelconque des revendications 1 à 14, dans lequel le canal comporte le passage qui est plus long que le seuil prédéterminé :
a) le passage étant sensiblement rectiligne ;
b) le seuil prédéterminé étant associé à une dimension de l'emballage de dispositif médical ; et/ou
c) le seuil prédéterminé étant proportionnel à une longueur du joint d'étanchéité perméable aux gaz.
